# EUROPEAN PATENT APPLICATION

(11) **EP 3 594 958 A1**
(43) Date of publication of application: **15.01.2020**
(21) Application number: 18763265.8
(22) Date of filing: 15.01.2018
(51) Int. Cl.: G16H 20/40

(54) **MEDICAL INFORMATION MANAGEMENT DEVICE, MEDICAL INFORMATION MANAGEMENT METHOD, AND MEDICAL INFORMATION MANAGEMENT SYSTEM**

(30) Priority: 07.03.2017 JP 2017042376
(71) Applicant: Sony Olympus Medical Solutions Inc., Tokyo 192-0904 (JP)
(72) Inventor: SUGAI, Toshiya, Hachioji-shi Tokyo 192-0904 (JP); ICHIKAWA, Kiyoshi, Hachioji-shi Tokyo 192-0904 (JP); TASHIRO, Hideki, Hachioji-shi Tokyo 192-0904 (JP); SUKIGARA, Ryu, Hachioji-shi Tokyo 192-0904 (JP)
(74) Representative: D Young & Co LLP
(86) International application number: PCT/JP2018/000851
(87) International publication number: WO 2018/163600

(57) **Abstract**

A medical information management apparatus including an integrated data index file generating unit that generates an integrated data index file in which an index indicating a storage location of surgical data which is various types of data acquired during surgery is integrated with an index indicating a storage location of patient information of a patient which is a subject of the surgery for each surgery is provided.

## Description

### Technical Field

The present disclosure relates to a medical information management apparatus, a medical information management method, and a medical information management system.

### Background Art

In surgery, various types of information (hereinafter also referred to as "surgical data") acquired during the surgery are recorded and stored. Examples of the surgical data include various types of video data (video data by an endoscope (endoscope video data), video data by a bird's eye view camera (bird's eye view camera video data), video data by a surgical camera (surgical camera video data), or the like), vital data of a patient (data of a pulse, blood pressure, a loss of blood volume, or the like of a patient), and log data of medical devices used for surgery (log data such as setting values when a pneumoperitoneum device, an energy device, or the like operates). The stored surgical data is used for the purpose of education of young doctors, documentation of conference presentation, or the like after completion of surgery.

Generally, these surgical data are recorded in different devices or servers in accordance with a type thereof. Therefore, in a case in which a doctor (user) desires to check these surgical data for a certain surgery all at once, it is necessary to access each device, each server, or the like and perform a task of extracting the surgical data at the time of the relevant surgery. Such a task is a heavy burden on the user.

In this regard, technology for managing surgical data efficiently has been developed. For example, a recording device that presets surgical data to be recorded and records the set surgical data in association with a time is disclosed in Patent Literature 1. Also, for example, an analysis device that inputs all of a plurality of types of surgical data recorded during surgery in one recording device and collectively manages them and displays a plurality of types of surgical data with the same time code is disclosed in Patent Literature 2. According to the techniques described in Patent Literatures 1 and 2, the doctor can check a plurality of types of surgical data displayed with the same time code without manually extracting the surgical data, and thus it is possible to reduce the burden on the user.

### Citation List

### Patent Literature

Patent Literature 1: WO 2006/077797 A
Patent Literature 2: WO 2006/077798 A

### Disclosure of Invention

### Technical Problem

However, in the method described in Patent Literature 1, since the preset surgical data is recorded, in a case in which the doctor desires to refer to other surgical data which is not set after the surgery, it is hard to cope with it. Also, since an amount of surgical data recorded during the surgery is huge, it is not realistic to store all piece of surgical data for certain surgery in one recording device as in the method described in Patent Literature 2. In particular, with the recent advance of video technology, the resolution of video captured during surgery is increasing, and the capacity of video data in surgical data is very large. In such a situation, it is more difficult to carry out the method described in Patent Literature 2.

In this regard, the present disclosure proposes a medical information management apparatus, a medical information management method, and a medical information management system which are novel and improved and capable of further improving the convenience of the user.

### Solution to Problem

According to the present disclosure, there is provided a medical information management apparatus including: an integrated data index file generating unit that generates an integrated data index file in which an index indicating a storage location of surgical data which is various types of data acquired during surgery is integrated, for each surgery, with an index indicating a storage location of patient information of a patient which is a subject of the surgery.

Moreover, according to the present disclosure, there is provided a medical information management method, including: generating, by a processor, an integrated data index file in which an index indicating a storage location of surgical data which is various types of data acquired during surgery is integrated, for each surgery, with an index indicating a storage location of patient information of a patient which is a subject of the surgery.

Moreover, according to the present disclosure, there is provided a medical information management system, including: a recording device that records and stores surgical data which is various types of data acquired during surgery during the surgery; a medical information management apparatus that manages the surgical data in an integrated manner; and a terminal that displays the surgical data, wherein the medical information management apparatus includes: an integrated data index file generating unit that generates an integrated data index file in which an index indicating a storage location of surgical data which is various types of data acquired during surgery is integrated, for each surgery, with an index indicating a storage location of patient information of a patient which is a subject of the surgery; a data acquiring unit that accesses, in a case in which a reference request for referring to the surgical data is input, a recording device in which the surgical data designated by the reference request is recorded using the integrated data index file corresponding to the surgical data designated by the reference request and acquires the surgical data designated by the reference request; and a presentation data generating unit that generates presentation data to be output in a terminal of a user who made the reference request using the surgical data acquired by the data acquiring unit.

According to the present disclosure, the integrated data index file in which the index indicating the storage location of the surgical data is integrated with the index indicating the storage location of the patient information for each surgery is generated. The integrated data index file does not include substantive content of the surgical data and the patient information but includes an index described therein, and thus it is easy to handle it. Therefore, it is easier to manage the surgical data and the patient information for each surgery using the integrated data index file. Therefore, it is possible to more easily read the surgical data and the patient information for the surgery after the surgery, and the convenience of the user can be improved.

### Advantageous Effects of Invention

As described above, according to the present disclosure, it is possible to further improve the convenience of the user. Note that the above effects are not necessarily limited, and in addition to or instead of the above effects, any of effects described in this specification or other effects which can be understood from this specification may be included.

### Brief Description of Drawings

FIG. 1 is a diagram illustrating an example of a configuration of a surgical data recording system according to the present embodiment.
FIG. 2 is a diagram conceptually illustrating a configuration of an integrated data index file.
FIG. 3 is a diagram illustrating an example of a configuration of a surgical data reference system according to the present embodiment.
FIG. 4 is a functional block diagram illustrating an example of a functional configuration of an index server.
FIG. 5 is a diagram illustrating an example of a display screen of a terminal on which surgical data is displayed.
FIG. 6 is a diagram illustrating another example of a display screen of a terminal on which surgical data is displayed.
FIG. 7 is a flowchart illustrating an example of a processing procedure of a medical information management method according to the present embodiment.
FIG. 8 is a block diagram illustrating an example of a hardware configuration of a medical information management apparatus according to the present embodiment.

### Best Mode for Carrying Out the Invention

Hereinafter, an exemplary embodiment of the present disclosure will be described in detail with reference to the appended drawings. In this specification and the drawings, components having substantially the same functional configuration will be assigned the same reference numerals, and redundant description thereof will be omitted.

The description will proceed in the following order.
1. Configuration of surgical data recording system
2. Configuration of surgical data reference system
3. Functional configuration of index server (medical information management apparatus)
4. Example of display screen
5. Medical information management method
6. Hardware configuration
7. Supplement

### (1. Configuration of surgical data recording system)

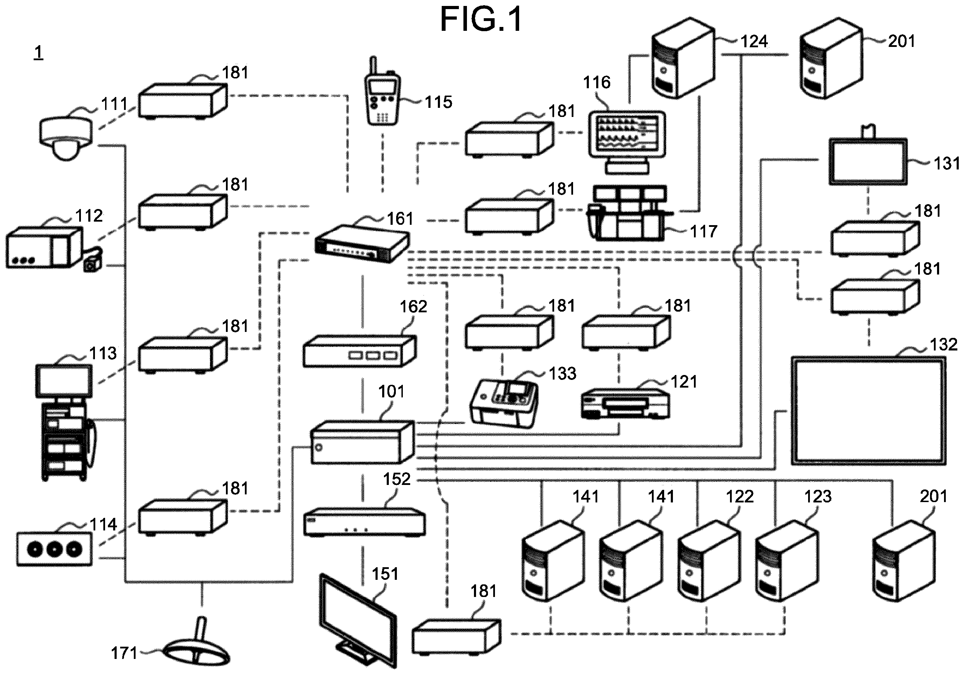

A configuration of a surgical data recording system according to an embodiment of the present disclosure will be described with reference to FIG. 1. FIG. 1 is a diagram illustrating an example of a configuration of a surgical data recording system according to the present embodiment. Note that the surgical data recording system corresponds to a medical information management system according to the present disclosure.

Referring to FIG. 1, a surgical data recording system 1 according to the present embodiment includes a medical controller 101, acquiring devices that acquire various types of surgical data (a bird's eye view camera 111, a surgical camera 112, an endoscope device/medical device 113, an external input terminal 114, an intercom 115, a biomonitor 116, and an anesthesia device 117), recording devices which records and stores the acquired surgical data (a recorder 121, a moving image server 122, a device log data server 123, and an anesthesiology server 124), patient information management apparatuses that manage information about patients (patient information) (an electronic medical chart server 141 and a digital imaging and communication in medicine (DICOM) server 142), data output devices that output the acquired surgical data and/or the patient information (ceiling-installed monitor 131, wall monitor 132, and printer 133), an operating device for operating the medical controller 101 (a touch panel 151), a switcher 161 that controls input/output of video data and/or audio data (hereinafter also referred to as video/audio data) in the acquired surgical data or display data of various types of information to/from each device, and an index server 201. Here, the index server 201 corresponds to a medical information management apparatus according to the present disclosure. In FIG. 1, the flow of information related to the video/audio data is indicated by a broken line, and the flow of information related to other information (for example, other surgical data, information related to device control, or the like) is indicated by a solid line.

The acquiring device, the data output device, and the operating device among these devices are installed in an operating room. Further, the medical controller 101, the recording device, the patient information management apparatus, the switcher 161, and the index server 201 are installed at a position outside the operating room, for example, an arbitrary position in a hospital or outside a hospital. However, the present embodiment is not limited to such an example, and the installation position of each device may be arbitrarily changed within the scope not affecting the surgery.

### (Medical controller)

The medical controller 101 is communicably connected to each of the above devices and controls an operation of each of these devices. However, in the illustrated configuration example, the medical controller 101 may not be connected to the intercom 115, the biomonitor 116, and the anesthesia device 117, and these devices may be able to operate independently of the medical controller 101.

Specifically, the medical controller 101 controls an operation of the acquiring device such that the acquiring device acquires the surgical data.

Also, the medical controller 101 controls an operation of the recording device such that the recording device records and stores the surgical data acquired by the acquiring device. At this time, the medical controller 101 controls an operation of the switcher 161 such that the video/audio data included in the surgical data is transmitted to the recording device via the switcher 161. Also, the medical controller 101 can access the recording device and the patient information management apparatus and read the surgical data and the patient information stored in these devices.

Also, the medical controller 101 controls an operation of the data output device such that the surgical data and/or the patient information are output from the data output device. Specifically, the medical controller 101 causes the video/audio data included in the currently acquired surgical data to be output from the data output device in accordance with an instruction of the user. Further, the medical controller 101 causes at least any one of display data of device log data, vital data, and anesthesia data included in the currently acquired surgical data and display data of electronic medical chart information and DICOM information included in the patient information to be output from the data output device in accordance with an instructions of the user. At this time, regarding the video/audio data and the display data of the device log data, the vital data, and the anesthesia data, the medical controller 101 controls an operation of the switcher 161 such that the video/audio data and the display data are transmitted to the data output device via the switcher 161. Note that in a case in which the data output device includes a plurality of devices as in the illustrated configuration example, different pieces of information may be output from the respective devices in accordance with an instruction of the user.

Further, the medical controller 101 can be connected with an illuminating device 171 that illuminates a surgical field with illumination light. The illuminating device 171 is, for example, a shadowless lamp. The medical controller 101 can also control an operation of the illuminating device 171 in accordance with an instruction of the user.

The medical controller 101 is constituted by, for example, a processor such as a central processing unit (CPU) or a digital signal processor (DSP). As the processor of the medical controller 101 executes arithmetic processing in accordance with a predetermined program, the functions described above are realized.

### (Operating device)

The operating device is an input interface that inputs various types of instructions to the medical controller 101. In a case in which the "operating device" is described in this specification, it refers to some or all of various types of devices for inputting various types of instructions to the medical controller 101 unless otherwise set forth herein. In the illustrated configuration example, the touch panel 151 is described as an example of the operating device. An instruction input from the touch panel 151 by the user is input to the medical controller 101 via a touch panel interface 152. The medical controller 101 controls operations of various types of connected devices in accordance with the instruction.

A type of operating device is not limited to the illustrated example, and various types of known input devices may be used as the operating device. For example, a mouse, keyboard, a switch, a lever, a remote-control device, or the like may be used as the operating device. Alternatively, the operating device may be a microphone or a camera, and the instruction may be input by a voice input through the microphone and/or a gesture input using a video captured by the camera. Also, the number of operating devices is not limited to the illustrated example, and a plurality of devices may be installed as the operating devices.

### (Acquiring device)

The acquiring device is a generic term for various types of devices that acquire the surgical data. In a case in which the "acquiring device" is described in this specification, it refers to some or all of various types of devices that acquire the surgical data unless otherwise set forth herein. In the illustrated configuration example, the acquiring device includes the bird's eye view camera 111, the surgical camera 112, the endoscope device/medical device 113, the external input terminal 114, the intercom 115, the biomonitor 116, and the anesthesia device 117.

The bird's eye view camera 111 is installed on the ceiling of the operating room and captures a situation in the operating room (particularly, a situation around an operating table on which the patient is located). The bird's eye view camera 111 acquires video data including the situation in the operating room as the surgical data.

The surgical camera 112 is installed near the operating table in the operating room and captures a situation of the surgical field. The surgical camera 112 acquires video data including the situation of the surgical field as the surgical data.

The endoscope device/medical device 113 includes an endoscope device and a medical device. The endoscope device/medical device 113 conceptually represents various types of devices used when treatment is performed on the patient as one device. Here, examples of the medical device include a pneumoperitoneum device that expands an abdominal cavity and creates a surgery space and an energy device that coagulates, incises, or seals tissue using energy such as high frequency current or ultrasonic vibration. However, the present embodiment is not limited to such an example, and the medical device may include various types of devices commonly used in surgery. The endoscope device acquires video data captured by an endoscope as the surgical data. Also, the medical device acquires log data of setting values (that is, the device log data) when it operates as the surgical data (for example, a setting value of an air supply amount if it is the pneumoperitoneum device, a setting value of output power if it is the energy device, and the like).

The external input terminal 114 is a terminal for receiving the video/audio data. As an external device is connected to the external input terminal 114, the video/audio data from the external device is input to the surgical data recording system 1.

The intercom 115 functions as a microphone and acquires audio data in the operating room as the surgical data. For example, the audio data is data about verbal instructions given by a supervisory doctor, conversations between medical staffs, or the like.

The biomonitor 116 acquires the vital data (for example, data such as pulse and blood pressure) of the patient in the surgery as the surgical data.

The anesthesia device 117 performs various types of controls on anesthesia during surgery such as administration of anesthesia to the patient and management of an anesthesia dose administered to the patient. The anesthesia device 117 acquires data related to anesthesia (the anesthesia data) such as the log data of the anesthesia device 117 as the surgical data in the process of performing various types of controls related to anesthesia. The anesthesia data includes, for example, data such as a setting value of an anesthesia dose administered to patient.

In the present embodiment, the wall monitor 132 may have a touch panel function. In this case, the wall monitor 132 also functions as the acquiring device, and information input through the wall monitor 132 can be the surgical data. For example, there are cases in which the supervisory doctor gives an instruction to the surgeon by writing information on the wall monitor 132 for the video displayed on the wall monitor 132 (for example, the video captured by the endoscope) during surgery. The information related to the instruction of the supervisory doctor input via the wall monitor 132 can be acquired as annotation data for the displayed video data as a part of the surgical data.

The video data acquired by the bird's eye view camera 111, the surgical camera 112, and the endoscope device of the endoscope device/medical device 113 are transmitted in accordance with a predetermined transmission scheme via a converter 181 and input to the switcher 161. Also, the video/audio data acquired by the external input terminal 114 is transmitted via the converter 181 in accordance with a predetermined transmission scheme and input to the switcher 161. Also, audio data acquired by the intercom 115 is transmitted via the converter 181 in accordance with a predetermined transmission scheme and input to the switcher 161. The switcher 161 transmits the video data, the video/audio data, and the audio data to the moving image server 122 via the converter 181 in accordance with a predetermined transmission scheme. At this time, in a case in which the annotation data is acquired, the annotation data is also transmitted from the switcher 161 to the moving image server 122 in association with corresponding video data. The moving image server 122 records and stores the data.

Also, the device log data acquired by the medical device of the endoscope device/medical device 113 is recorded and stored in the device log data server 123 via the medical controller 101. Further, the display data of the device log data acquired by the medical device is transmitted to the switcher 161 via the converter 181 in accordance with a predetermined transmission scheme.

Also, the vital data and the anesthesia data acquired by the biomonitor 116 and the anesthesia device 117 are recorded and stored in the anesthesiology server 124. Further, the display data of the vital data and the display data of the anesthesia data acquired by the biomonitor 116 and the anesthesia device 117 are transmitted to the switcher 161 via the converter 181 in accordance with a predetermined transmission scheme. As described above, since the biomonitor 116 and the anesthesia device 117 are installed independently of the medical controller 101, the vital data and the anesthesia data acquired by the biomonitor 116 and the anesthesia device 117 are directly recorded and stored in the anesthesiology server 124 without going through the medical controller 101, and the display data of the vital data and the display data the anesthesia data are directly transmitted to the switcher 161.

Further, the acquiring device can simultaneously acquire a time at which the surgical data is acquired in association with the surgical data when acquiring the surgical data. In other words, time information is assigned to the acquired surgical data as meta data.

Also, the configuration of the illustrated acquiring device is merely an example, and the acquiring device may include other devices not illustrated in FIG. 1. For example, the acquiring device may include other medical devices not illustrated in FIG. 1, and log data acquired by the other medical device may be acquired as the device log data. Also, for example, the loss of blood volume of the patient or the infusion volume administered to the patient can be measured and recorded at predetermined time intervals during the surgery. Such information may also be acquired as the surgical data in association with the time information and recorded and stored in any one of the recording devices. For example, data for the loss of blood volume and the infusion volume can be recorded and stored in the anesthesiology server 124 as the vital data.

### (Recording device)

The recording device is a generic term for various types of devices that record and store the surgical data. In a case in which the "recording device" is described in this specification, it refers to some or all of various types of devices that record and store the surgical data unless otherwise set forth herein. In the illustrated configuration example, the recording device includes the recorder 121, the moving image server 122, the device log data server 123, and the anesthesiology server 124.

The moving image server 122 records and stores the video/audio data among the surgical data. Specifically, the moving image server 122 receives, records, and stores the video/audio data acquired by the video/audio data acquired by the bird's eye view camera 111, the surgical camera 112, the endoscope device/medical device 113, the external input terminal 114, and the intercom 115 via the switcher 161. At this time, in a case in which the video/audio data acquired by the acquiring device includes the annotation data by the supervisory doctor, the medical controller 101 records and stores the annotation data in the moving image server 122 in association with the video/audio data.

The device log data server 123 receives the device log data acquired by the medical device of the endoscope device/medical device 113 among the surgical data, through the medical controller 101 and records and stores the device log data.

The anesthesiology server 124 records and stores the vital data and the anesthesia data acquired by the biomonitor 116 and the anesthesia device 117 among surgical data.

The recorder 121 functions as a backup of the moving image server 122, the device log data server 123, and the anesthesiology server 124. The recorder 121 records and stores part or all of the surgical data recorded and stored in the moving image server 122, the device log data server 123, and the anesthesiology server 124. A type of surgical data recorded and stored in the recorder 121 may be appropriately decided by the user. As the recorder 121 is installed, the reliability of the surgical data recording system 1 is improved since the surgical data is recorded and stored even if which an error occurs in the moving image server 122, the device log data server 123, or the anesthesiology server 124. In the illustrated configuration example, only one recorder 121 is installed, but a plurality of recorders 121 may be installed.

All of the recorder 121, the moving image server 122, the device log data server 123, and the anesthesiology server 124 record and store the surgical data in association with the time at which the surgical data is acquired. Also, the electronic medical chart information of the patient who is currently undergoing the surgery which is stored in the electronic medical chart server 141 of the patient information management apparatus may be provided to the recorder 121, the moving image server 122, the device log data server 123, and the anesthesiology server 124 under the control of the medical controller 101, and the recorder 121, the moving image server 122, the device log data server 123, and the anesthesiology server 124 may record and store the surgical data in association with the electronic medical chart information (for example, in association with a patient ID, an ID of a surgeon who is doing surgery, or the like).

### (Patient information management apparatus)

The patient information management apparatus is a generic term for various types of devices that manage the patient information. In a case in which the "patient information management apparatus" is described in this specification, it refers to some or all of various types of devices that manage the patient information unless otherwise set forth herein. In the illustrated configuration example, the patient information management apparatus includes the electronic medical chart server 141 and the DICOM server 142.

The electronic medical chart server 141 stores the electronic medical chart information of the patient. The electronic medical chart information includes a patient ID identifying the patient, body data of the patient, a medical history of the patient, a past surgery history of the patient, and the like. Further, the surgery history may include an ID identifying the surgeon who was in charge of the surgery, a nurse who participated in the surgery, and the like. Further, the electronic medical chart information may include various types of information generally included in the electronic medical chart information.

The DICOM server 142 stores DICOM information of the patient. The DICOM information includes CT image data of the patient, MRI image data, and the like. The DICOM server 142 manages the DICOM information in association with the patient ID. Further, the DICOM information may include various types of information generally included in the DICOM information.

### (Switcher)

The switcher 161 controls input/output of the video/audio data and the display data to/from each device. In the surgical data recording system 1, the video/audio data and the display data are input and output to various types of devices via the switcher 161. The operation of the switcher 161 is controlled by a switcher control device 162. The medical controller 101 can control the operation of the switcher 161 via the switcher control device 162.

Specifically, as described above, the switcher 161 transmits the video/audio data acquired by the acquiring device to the moving image server 122 via the converter 181 in accordance with a predetermined transmission scheme. Also, the switcher 161 acquires the surgical data acquired by the acquiring device via the converter 181 in accordance with a predetermined transmission scheme, and transmits the surgical data to the data output device via the converter 181 in accordance with an instruction from the medical controller 101. Also, the switcher 161 acquires the electronic medical chart information and the DICOM information stored in the patient information management apparatus in accordance with an instruction from the medical controller 101, and transmits the display data to the data output device via the converter 181 in accordance with a predetermined transmission scheme.

In the surgical data recording system 1, a plurality of transmission paths corresponding to different transmission formats may be installed as transmission paths of the video/audio data and the display data. Accordingly, the video/audio data and the display data can be transmitted normally via other communication paths even if an abnormality occurs in one communication path. As the transmission scheme, for example, a digital visual interface (DVI), a serial digital interface (SDI) (for example, an HD-SDI), or the like may be used. Also, Internet protocol (IP) transmission may be used. In a case in which a plurality of transmission paths corresponding to different transmission formats are installed, devices corresponding to transmission formats employed therein are installed as the external input terminal 114, the switcher 161 and the converter 181 (that is, a plurality of external input terminals 114, a plurality of switchers 161, and a plurality of converters 181 are installed).

### (Data output device)

The data output device is a generic term for various types of devices that output at least the acquired surgical data. In a case in which the "data output device" is described in this specification, it refers to some or all of various types of devices that output at least the acquired surgical data unless otherwise set forth herein. The data output device may display the patient information in addition to the surgical data. In the illustrated configuration example, the data output device includes the ceiling-installed monitor 131, the wall monitor 132, and the printer 133.

The ceiling-installed monitor 131 is a monitor which is installed on the ceiling in the operating room. The wall monitor 132 is a monitor installed on the wall in the operating room. For example, the ceiling-installed monitor 131 and the wall monitor 132 display the video data included in the surgical data. Note that each of the ceiling-installed monitor 131 and the wall monitor 132 may include a speaker and output audio data included in the surgical data together with the video data. Also, for example, the ceiling-installed monitor 131 and the wall monitor 132 display the device log data, the vital data, and/or the anesthesia data included in the surgical data in the form of a graph along a time axis. Also, for example, the ceiling-installed monitor 131 and the wall monitor 132 display the electronic medical chart information and/or the DICOM information included in the patient information in the form of a table, an image, or the like.

Types of the ceiling-installed monitor 131 and the wall monitor 132 are not limited, and various types of known display devices such as a liquid crystal display device, a plasma display device, or an organic electroluminescence (EL) display device may be used as these monitors.

The printer 133 prints the surgical data and/or the patient information on paper in the form of an image (a still image), a graph, or the like.

### (Index server 201)

The index server 201 is configured to be accessible to the recording device and the patient information management apparatus. The index server 201 mainly has two functions. A first function is a function of generating and storing an index file for reading the surgical data recorded in the recording device and the patient information stored in the patient information management apparatus. A second function is a function of reading the surgical data and/or the patient information corresponding to a reference request from the user from the recording device and/or the patient information management apparatus on the basis of the index file generated by the user and generating presentation data to be presented to the user on the basis of the surgical data and the patient information. The first function will be mainly described here since the second function will be described in (2. Configuration of surgical data reference system) again.

A specific device configuration of the index server 201 may be similar to that of a common server. The index server 201 includes a processor such as a CPU or DSP and a storage element such as a memory. The above functions are realized as the processor of the index server 201 executes arithmetic processing in accordance with a predetermined program.

In the configuration example illustrated, the index server 201 is communicably connected to the moving image server 122, the device log data server 123, and the anesthesiology server 124 included in the recording device and the electronic medical chart server 141 and the DICOM server 142 included in the patient information management apparatus (in FIG. 1, two index servers 201 are illustrated for convenience of drawing, but only one the index server 201 is actually installed).

If certain surgery is completed, the index server 201 generates an index file for the surgical data acquired in the middle of the surgery and the patient information for the patient who is the subject of the surgery. In other words, the index file is generated for each surgery. In this specification, the index file corresponding to one surgery is also referred to as an "integrated data index file."

The integrated data index file is a file in which information (index) indicating a storage location of the surgical data and the patient information for certain surgery is described. For example, the index can be an address indicating the storage location of the surgical data and the patient information. By using the integrated data index file, it is possible to easily read the surgical data and the patient information for the surgery from the recording device and the patient information management apparatus after the surgery is completed.

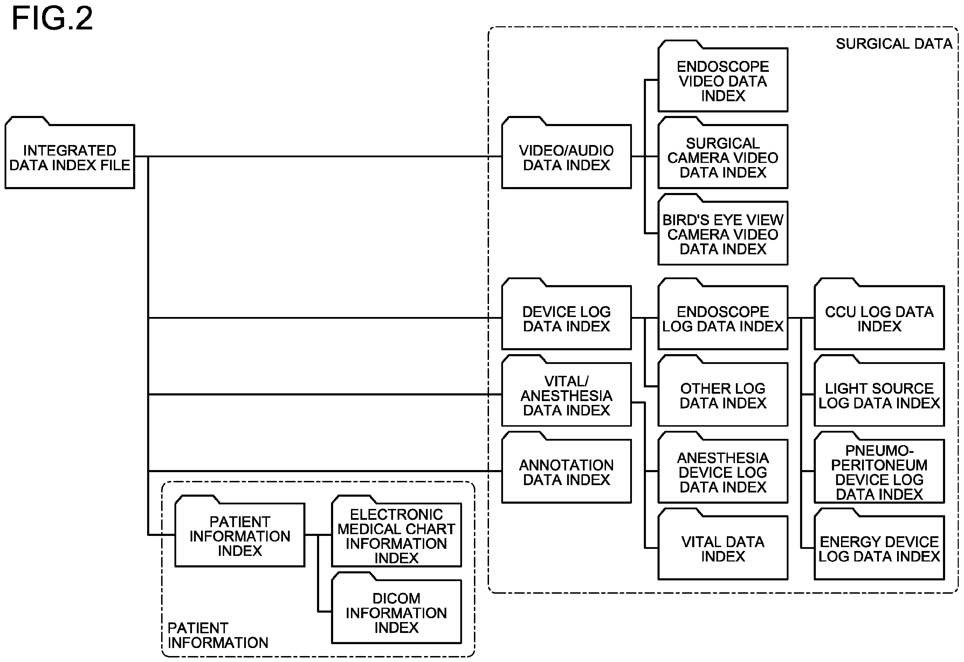

FIG. 2 is a diagram conceptually illustrating a configuration of the integrated data index file. As illustrated in FIG. 2, the integrated data index file hierarchically includes indexes (addresses indicating a storage location of each piece of data) for accessing the surgical data and the patient information. For example, the integrated data index file includes a video/audio data index (an index for accessing the video/audio data in the moving image server 122), a device log data index (an index for accessing the device log data in the device log data server 123), a vital/anesthesia data index (an index for accessing the vital data and the anesthesia data in the anesthesiology server 124), an annotation data index (an index for accessing the annotation data in the moving image server 122), and a patient information index (an index for accessing the electronic medical chart information in the electronic medical chart server 141 and the DICOM information in the DICOM server 142). Note that the surgical data indicated by the video/audio data index, the device log data index, the vital/anesthesia data index, and the annotation data index is the surgical data acquired with the same time code in the same surgery.

Further, the video/audio data index includes an endoscope video data index (an index for accessing endoscope video data in the video/audio data), a surgical camera video data index (an index for accessing surgical camera video data in the video/audio data), and a bird's eye view camera video data index (an index for accessing bird's eye view camera video data in the video/audio data).

Further, the device log data index includes an endoscope log data index (an index for accessing log data acquired by the endoscope device/medical device 113 in the device log data) and other log data indexes (indexes for accessing log data of other medical devices (not illustrated in FIG. 1) in the device log data). Further, the endoscope log data index includes a CCU log data index (an index for accessing log data of a camera control unit (CCU) in the log data acquired by the endoscope device/medical device 113), a light source log data index (an index for accessing log data of the light source in the log data acquired by the endoscope device/medical device 113), a pneumoperitoneum device log data index (an index for accessing log data of the pneumoperitoneum device in the log data acquired by the endoscope device/medical device 113), and an energy device log data index (an index for accessing log data of the energy device in the log data acquired by the endoscope device/medical device 113).

Further, the vital/anesthesia data index includes an anesthesia device log data index (an index for accessing log data acquired by the anesthesia device 117 in the anesthesia data) and a vital data index (an index for accessing log data acquired by the biomonitor 116).

Further, the patient information index includes an electronic medical chart information index (an index for accessing the electronic medical chart information in the electronic medical chart server 141) and a DICOM information index (an index for accessing the DICOM information in the DICOM server 142).

Here, in general, in a hospital system, the surgical data is recorded and stored in a different device or server depending on a type. Also, the surgical data and the patient information are stored in different devices. Therefore, if the user desires to refer to the surgical data and the patient information for certain surgery is made, it is necessary for the user to manually access the respective devices and extract necessary data, leading to a heavy work burden.

The surgical data recording system 1 illustrated in FIG. 1 is similar to a configuration of such a common system in that the surgical data and the patient information are recorded and stored in different devices (the recording device and the patient information management apparatus). However, in the present embodiment, the index server 201 capable of accessing the recording device and the patient information management apparatus is installed as described above. Further, the index server 201 generates the integrated data index file for each surgery. Because the surgical data and the patient information can be managed in an integrated manner for each surgery on the basis of integrated data index file, the user can easily access the surgical data and the patient information of each surgery using the integrated data index file without performing the task of manually extracting data as described above. Therefore, the work burden on the user can be reduced, and the convenience of the user can be improved.

Further, the surgical data recording system 1 can be constituted only by newly installing the index server 201 in the common system. Therefore, the surgical data recording system 1 can be constituted without changing the existing system significantly. Therefore, the cost for constructing the surgical data recording system 1 can be kept relatively small.

It should be noted that integrated data index file is not a file containing substantive content of the surgical data and the patient information but a file in which information for accessing the surgical data and the patient information is described. For example, the method described in Patent Literature 2 is considered to be employed as the method for managing the surgical data and the patient information in an integrated manner. In this method, the surgical data and the patient information are managed in an integrated manner as the surgical data and the patient information are recorded and stored in a single recording device for each surgery. However, the surgical data and the patient information generated in one surgery are huge, and a data amount thereof is very large. In particular, in recent years, the resolution of video data has been increased to, for example, 4K, and the capacity of video data in the surgical data has been increasing. In such a situation, it is likely that it is difficult to record and store the surgical data and the patient information in a single recording device for each surgery as in the method described in Patent Literature 2.

On the other hand, in the present embodiment, the surgical data and the patient information are managed by generating the integrated data index file as described above. The integrated data index file is a file in which the surgical data and the patient information are virtually integrated, and it does not contain the substantive content of the surgical data and the patient information, and the capacity thereof is not very large. Since it is possible to manage the surgical data and the patient information indirectly in an integrated manner by managing the integrated data index file, according to the present embodiment, it is possible to more easily manage the surgical data and the patient information.

The configuration of the surgical data recording system 1 has been described above. The configuration of the surgical data recording system 1 illustrated in FIG. 1 is merely an example, and the configuration of the surgical data recording system 1 can be arbitrarily changed. As described above, the surgical data recording system according to the present embodiment can be constituted by installing the index server so that the recording device and the patient information management apparatus can be accessed in the existing common hospital system. In other words, the surgical data recording system according to the present embodiment may have a configuration in which the index server is additionally installed to various types of known hospital systems.

For example, depending on a hospital system, the device log data may be recorded and stored in each medical device instead of being recorded and stored in the device log data server 123 in an integrated manner. In a case in which the surgical data recording system according to the present embodiment is constituted using such a system, the device log data server 123 may not be installed, and each medical device may function as both the acquiring device and the recording device.

Also, the index server 201 may detect an event related to surgery from the surgical data recorded in any one of the recording devices and generate event information in association with the surgical data. The event related to the surgery includes, for example, either or both of a change in a state of a surgery subject and an operating status of the surgical device. Also, the event information is information related to the event which occurred, and the event information includes, for example, a surgical data type, an event type, and an event occurrence time.

Specifically, for example, the index server 201 detects events related to a "use status of the energy device based on the log data (for example, a excision time and a excision frequency of an electric scalpel, a use frequency of a monopolar, or the like)," a "bleeding time and a bleeding situation based on the basis of the video data or the vital data of the endoscope or the surgical camera," a "speech situation (for example, a surprised voice, an angry voice, pointing-out, or the like) or speech content of surgery related parties based on the audio data," a "situation in an operating room based on the video data of the bird's eye view camera," and the like and generates the event information associated with the corresponding surgical data. For example, the generated event information is stored in the index server 201 so that it can be referred to from a surgical data reference system.

Also, the index server 201 may be configured not to generate the event information, but the acquiring device may detect an event from the acquired surgical data and record and store the event information related to the detected event in any one of the recording devices in association with the surgical data.

Devices generally used in the hospital system can be used as devices constituting the surgical data recording system 1 illustrated in FIG. 1. Therefore, a specific device configuration of each device is omitted here.

### (2. Configuration of surgical data reference system)

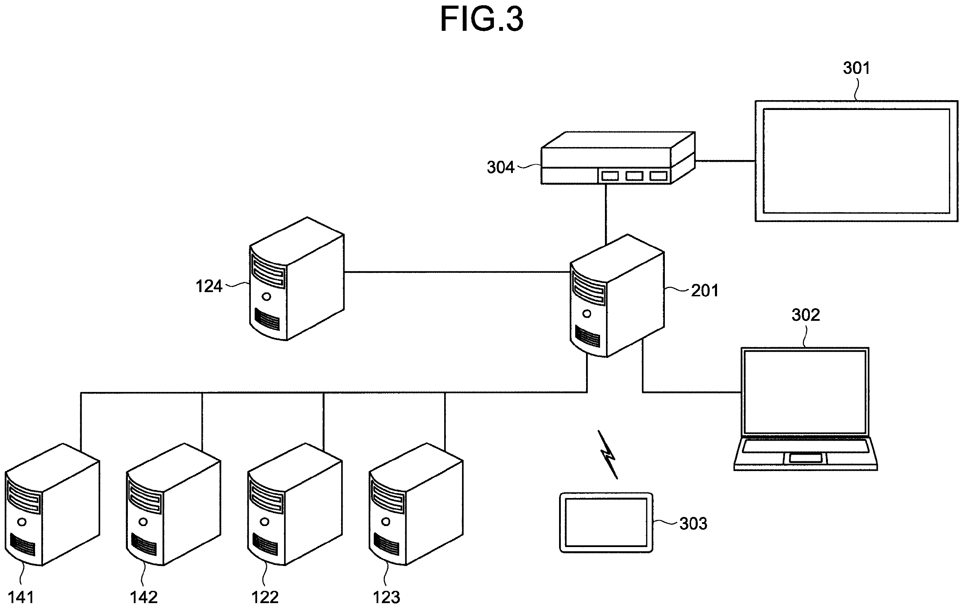

A configuration of a surgical data reference system according to the present embodiment will be described with reference to FIG. 3. FIG. 3 is a diagram illustrating an example of a configuration of the surgical data reference system according to the present embodiment. The surgical data reference system is a system that enables the user to refer to the surgical data using the integrated data index file generated in the surgical data recording system 1 illustrated in FIG. 1. The surgical data reference system corresponds to a medical information management system according to the present disclosure.

Referring to FIG. 3, a surgical data reference system 2 according to the present embodiment mainly includes the index server 201, the recording device (the moving image server 122, the device log data server 123, and the anesthesiology server 124), the patient information management apparatus (the electronic medical chart server 141 and the DICOM server 142), and a terminal that presents at least the surgical data to the user (a conference monitor 301, a medical office personal computer (PC) 302, and a tablet PC 303). The recording device (the moving image server 122, the device log data server 123, and the anesthesiology server 124) and the patient information management apparatus (the electronic medical chart server 141 and the DICOM server 142) have already been described above with reference to FIG. 1, and thus description thereof is omitted here.

The terminal is a generic term for various types of devices that present various types of information including the surgical data to the user. In a case in which the "terminal" is described in this specification, it refers to some or all of various types of devices that present various types of information including the surgical data to the user unless otherwise set forth herein. In the illustrated example, the terminal includes the conference monitor 301, the medical office PC 302, and the tablet PC 303.

The conference monitor 301 is a monitor installed in a conference room in a hospital. The conference monitor 301 is connected to the index server 201 via a setup box 304 via an in-hospital network and outputs presentation data generated by the index server 201. A type of the conference monitor 301 is not limited, and various types of known display devices such as a liquid crystal display device, a plasma display device, or an organic EL display device may be used as the conference monitor 301.

The medical office PC 302 is a PC installed in a medical office in a hospital. The medical office PC 302 is connected to the index server 201 via the in-hospital network and outputs the presentation data generated by the index server 201.

The tablet PC 303 is a tablet PC owned by a hospital staff. The tablet PC 303 is connected to the index server 201 via the in-hospital network and outputs the presentation data generated by the index server 201. The tablet PC 303 may be taken out of the hospital.

The presentation data includes at least display screen data to be displayed on a display screen of the terminal. In the terminal, the display screen data is displayed on the display screen. Also, the presentation data may further include audio data output from the terminal, and the audio data may be output in the terminal. The output of the presentation data may be executed using software installed in the terminal in advance, or dedicated application software for outputting the presentation data to the terminal may be provided.

The index server 201 is identical to the index server 201 illustrated in FIG. 1. In the surgical data reference system 2, the index server 201 executes the above-described second function (the function of reading the surgical data and/or the patient information corresponding to the reference request from the user on the basis of the index file generated by the index server 201 from the recording device and/or the patient information management apparatus and generating the presentation data to be presented to the user on the basis of the surgical data and/or the patient information which is read).

Specifically, in the surgical data reference system 2, a request (reference request) for referring to the surgical data is input from the user to the index server 201. The reference request includes at least information specifying the surgical data which the user desires to refer to. Specific content of the information specifying the surgical data is not limited. For example, the reference request may be a request of specifically pointing out specific surgical data which the user desires to refer to. Alternatively, the reference request may be a keyword specifying the surgical data which the user desires to refer to. The keyword may be, for example, a patient ID of a patient who was the subject of the surgery, an ID of a surgeon who performed the surgery, an ID of a medical staff who participated in the surgery, a date and time of the surgery, a location of the surgery, and/or the like. With the reference request, only one type of surgical data may be requested, or a plurality of types of surgical data may be requested. In a case in which a plurality of types of surgical data are required, such surgical data may be surgical data in a plurality of different surgeries.

The input of the reference request can be performed via the terminal. In this case, application software for inputting the reference request to the terminal may be provided. Alternatively, the reference request may be directly input via an input device installed in the index server 201.

A graphical user interface (GUI) that enables the user to input the reference request may be provided. In the GUI, for example, a template in which a type of surgical data to be referred is specified in advance may be prepared in accordance with the purpose of referring to the surgical data. If the template is used, since the type of surgical data is specified in advance, the user only needs to designate the surgery in which the surgical data is referred to, and thus the operability of the user is improved. For example, for the educational purposes, an input screen for designating two different pieces of endoscope video data may be displayed as the GUI. In the input screen, for example, the user designates endoscope video data at the time of surgery performed by the user as one endoscope video data using the above-described keyword and designates endoscope video data at the time of surgery for the same case performed by the supervisory doctor as the other endoscope video data. In this case, since these two pieces of endoscope video data are read, the user can look back on his/her own technique with reference to these endoscope video data while comparing them. Alternatively, for example, if the purpose is documentation for conference presentation, an input screen for designating surgical data frequently included in materials for conference presentation using a keyword or the like may be displayed as the GUI.

Also, in the present embodiment, the reference request also includes a request for a display screen layout when the surgical data requested to be referred to is displayed on the terminal. Such a request specifically includes information regarding how to divide an area in a screen, a type of surgical data to be displayed in each area, and the like. A dedicated GUI for inputting a request for such a display screen layout may be provided.

The reference request may further include information specifying the patient information to be referred to in addition to the information specifying the surgical data to be referred to. The information specifying the patient information may be information of pointing out specific patient information, similarly to the information specifying the surgical data or may be a keyword specifying the patient information to be referred to. Also, only one piece of patient information may be requested, or a plurality of types of patient information may be requested. Further, the GUI and the template may be provided for the patient information.

The index server 201 extracts the integrated data index file corresponding to the input reference request from among the integrated data index files generated and stored for each surgery (that is, the integrated data index file for the surgical data specified by the input reference request). Then, the index server 201 accesses the recording device using the extracted integrated data index file and acquires the surgical data corresponding to the input reference request. In a case in which the reference request includes the information specifying the patient information to be referred to, the index server 201 similarly extracts integrated data index file for the patient information, accesses the patient information management apparatus using the extracted integrated data index file, and acquires the patient information corresponding to the input reference request.

If the surgical data is acquired, the index server 201 generates the presentation data which is data to be presented to the user. For example, the index server 201 generates display screen data in which the surgical data is placed on the display screen in accordance with the request for the display screen layout included in the reference request as the presentation data. In a case in which the reference request includes the information specifying the patient information to be referred to, and the patient information is also acquired, the index server 201 generates the display screen data including the patient information.

Then, the index server 201 transmits the generated display screen data to the terminal as the presentation data. As the transmitted display screen data is displayed on the terminal, the user can refer to the surgical data (and the patient information) requested by the user via the terminal. In a case in which reference to the audio data included in the surgical data is also requested by the reference request, the presentation data also includes the audio data.

Further, in a case in which the requested surgical data includes video data, the terminal may collectively download the video data from the index server 201 and then play back the video data, or the index server 201 may deliver the video data in a streaming manner. However, as described above, the capacity of the video data is large especially in a case in which shooting is performed with a high resolution, and thus it is desirable to use streaming delivery, depending on the specifications of the terminal, in order to execute the playback of the video data smoothly.

Also, in a case in which the requested surgical data includes a plurality of types of surgical data for the same surgery, the index server 201 may display these surgical data with the same time code in the display screen data. Accordingly, the user can refer to a plurality of types of surgical data on the same time axis corresponding to the progress of the surgery.

Also, in a case in which the requested surgical data includes the video data, the index server 201 may display a group of thumbnail images of the video data in the display screen data. The group of thumbnail images may be one in which thumbnail images at respective times at which the video data is divided at predetermined time intervals are arranged chronologically in a predetermined direction in the display screen. The video data can be played back from the time corresponding to the selected thumbnail image when one thumbnail image in the group of thumbnail images is selected in the display screen data. In other words, the user can designate a playback position of the video data using a group of thumbnail images. Accordingly, the user can refer to the video data from a desired time, and thus the convenience of the user is improved. In particular, when the video data at the time of his/her own surgery is compared with the video data at the time of surgery by the supervisory doctor for educational purposes, since these are video data acquired at the time of different surgeries, naturally, they are not video data which are acquired with the same time code. Therefore, since a group of thumbnail images is prepared, and the user can appropriately designate the playback position of each piece of video data, the user can refer to the video data acquired with different time codes while comparing them by performing, for example, an operation of playing back the respective pieces of video data simultaneously from the position for the same technique.

Further, in a case in which the requested surgical data includes the video data, the index server 201 may display the video data in the display screen data with the reduced resolution. Since the data capacity of the display screen data can be reduced by reducing the resolution, it is easier to transmit the display screen data to the terminal and to refer to the display screen data in the terminal. In this case, when the user performs a predetermined operation (for example, an operation to select an area in which the video data is displayed or the like), the display screen data may be changed, and the high-resolution video data may be displayed again.

Further, the index server 201 may store the generated previous presentation data. Then, when the user inputs the reference request, the previous presentation data is presented to the user, and the user may be able to select the previous presentation data and input the reference request corresponding to the presentation data. Accordingly, it is possible to input the reference request with a simpler operation without having to input the reference request from the beginning again in a case in which it is desired to refer to the display screen layout and the combination of the surgical data that the user referred to in the past, and thus the convenience of the user is improved.

The configuration of the surgical data reference system 2 according to the present embodiment has been described above. The configuration of the surgical data reference system 2 is not limited to the illustrated example. In the surgical data reference system 2, the index server 201 may be communicably connected to the recording device and the terminal, and specific types of recording device and terminal to be connected may be decided appropriately. For example, in a case in which some of the surgical data which is not allowed to be referred to by the user is decided in advance, the recording device in which the surgical data is recorded and stored may not be included in the surgical data reference system 2. Also, for example, the type of terminal is not limited to the illustrated example, and various types of devices may be used as the terminal as long as it is a device having a display function. For example, a monitor in an operating room such as the wall monitor 132 illustrated in FIG. 1 may function as the terminal. In this case, when the reference request is transmitted to the index server 201 during surgery, it is possible to immediately check the surgical data in the past surgery.

As described above, according to the surgical data reference system 2, desired surgical data can be selected by a simple operation of inputting the reference request, and thus the convenience of the user can be improved. At this time, it is possible to designate a plurality of types of surgical data acquired in the same surgery or acquired in different surgeries by a simple operation.

Therefore, for example, it is easy to refer to different types of surgical data with the same time code. Further, for example, it is possible to easily compare the video data in the surgery performed by the user with the video data in the surgery for the same case performed by the supervisory doctor. At this time, according to the surgical data reference system 2, since the user can freely set the display screen layout when the surgical data to be referred to is displayed on the terminal, for example, it is possible to compare the surgical data and the video data with a desired layout. The example of the display screen displayed on the terminal will be described in (4. Example display screen).

Here, usefulness of surgery video in training of doctors, especially, surgeons is generally recognized, and educational effects of watching of a video with an audio can also be seen in various conference presentations or the like. Further, regarding the educational effects by this video, there is an opinion that the educational effects can be further increased by displaying both the endoscopic video and the video outside the body cavity, especially, in the endoscopic surgery together and viewing these videos related to manipulations of forceps in parallel. Also, in general, for the purpose of education, young doctors are watching surgery videos of excellent surgeons and imitating the technique. At this time, it is effective to view the surgery videos in parallel in order to understand a difference between his/her own surgery and the surgery of the excellent surgeon.

As described above, in the medical field, there has been a great demand for a technique of enabling the user to view his/her own surgery video and other videos on a single screen simultaneously. However, with the existing techniques, it is difficult to manage the surgical data in an integrated manner, and thus it was difficult to satisfy such a demand. For example, it is necessary for the user to manually perform a task of specifying devices that store his/her surgery video and other videos that the user desires to refer to and extracting the video data from the devices, and the burden is great. Further, even when the two extracted videos are compared, it is necessary to display the two videos separately and view the two videos alternately, and it is difficult to compare the videos.

On the other hand, in the surgical data reference system 2, as described above, it is possible to select a plurality of pieces surgical data which the user desires to refer to with a simple operation, and the user can freely set the display screen layout of the surgical data, and thus the above-described desire of viewing two different surgery videos while comparing them can be appropriately satisfied. As described above, the surgical data reference system 2 can be a very useful system for teaching young doctors.

Furthermore, for example, it is very useful to improve the quality of surgery if it is possible to display the surgery video of the excellent surgeon during surgery. A navigation system is widely used as a system to support surgery, but the navigation system displays a procedure of a technique for virtual organs drawn by computer graphics (CG) or the like but does not display a video which is to become an example corresponding to a corresponding scene. According to the surgical data reference system 2, as described above, the video data at the time of the past surgery can be displayed on the monitor in the operating room during the surgery with a simple operation, and thus it is possible to provide useful information as navigation at the time of surgery.

### (3. Functional configuration of index server (medical information management apparatus))

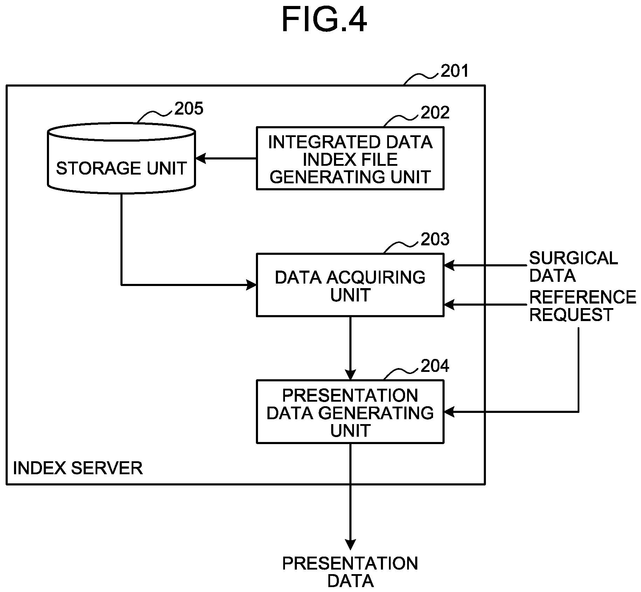

A functional configuration of the index server 201 illustrated in FIGS. 1 and 3 will be described with reference to FIG. 4. FIG. 4 is a functional block diagram illustrating an example of a functional configuration of the index server 201.

Referring to FIG. 4, the index server 201 includes an integrated data index file generating unit 202, a data acquiring unit 203, a presentation data generating unit 204, and a storage unit 205 as its functions. Among these functions, the integrated data index file generating unit 202, the data acquiring unit 203, and the presentation data generating unit 204 can be realized as the processor of the index server 201 executes arithmetic processing in accordance with a predetermined program. Further, the storage unit 205 is realized by a storage device such as a hard disc drive (HDD).

The integrated data index file generating unit 202 generates the integrated data index file for each surgery. In other words, the integrated data index file generating unit 202 corresponds to the function of the index server 201 in the above-described surgical data recording system 1. The integrated data index file generating unit 202 stores the generated integrated data index file in the storage unit 205.

A trigger that causes the integrated data index file generating unit 202 to generate the integrated data index file may be set arbitrarily. For example, the integrated data index file generating unit 202 may detect that the surgery has been performed in accordance with an instruction given by the user after the surgery is completed and generate the integrated data index file or may detect that the surgery has been performed by monitoring the recording devices one after another or detecting that new surgical data has been recorded in the recording device and generate the integrated data index file.

The data acquiring unit 203 extracts the integrated data index file corresponding to the reference request from the storage unit 205 in accordance with the reference request input from the user, accesses the recording device using the extracted integrated data index file, and acquires the surgical data designated by the reference request. In a case in which the request for the patient information is also included in the reference request, the data acquiring unit 203 similarly extracts the integrated data index file corresponding to the reference request from the storage unit 205, accesses the patient information management apparatus using the integrated data index file, and acquires the patient information designated by the reference request.

The data acquiring unit 203 provides the acquired surgical data to the presentation data generating unit 204. In a case in which the patient information is acquired, the data acquiring unit 203 also provides the acquired patient information to the presentation data generating unit 204.

The presentation data generating unit 204 generates the presentation data to be presented to the user in accordance with the reference request input from the user using the surgical data obtained by the data acquiring unit 203. The reference request includes the information about the display screen layout, and the presentation data generating unit 204 generates display screen data as the presentation data so that the display screen layout is realized. In a case in which the patient information is acquired, the presentation data generating unit 204 generates the display screen data using the patient information as well. Also, in a case in which the reference request includes a request for audio data, the presentation data generating unit 204 also includes the audio data in the presentation data.

Further, the presentation data generating unit 204 may include the event information related to the surgical data included in the presentation data in the presentation data. For example, the presentation data generating unit 204 extracts the event information related to the event associated with the surgery detected from the surgical data on the basis of an instruction of the user. Then, the presentation data generating unit 204 generates the extracted event information as the presentation data. The presentation data corresponding to the extracted event information may include other information.

Further, the presentation data generating unit 204 may cause the event information corresponding to the surgical data to be displayed in accordance with the time of the surgical data displayed on the screen. Further, the presentation data generating unit 204 may add corresponding event information to each thumbnail image and cause the event information to be displayed. Furthermore, the presentation data generating unit 204 may search for the event information on the basis of an instruction of user and present the corresponding surgical data.

The presentation data generating unit 204 transmits the generated presentation data to the terminal. In the terminal, the presentation data is displayed and viewed by the user.

The example of the functional configuration of the index server 201 has been described above. The specific device configuration of the index server 201 is not limited. Each component illustrated in FIG. 4 may be constituted using a general-purpose member or may be constituted by hardware specialized for the function of each component. Such a configuration may be changed appropriately depending on the level of technology at the time of implementation.

Also, the index server 201 may not necessarily be one device and may be realized by cooperation of a plurality of devices. In this case, the functions of the index server 201 are distributed and installed in a plurality of devices having a configuration capable of realizing these functions, and the function of the index server 201 as a whole can be realized as a plurality of devices cooperate with one another while exchanging various types of information.

Further, it is possible to generate a computer program for realizing the functions of the index server 201 described above and install it in a PC or the like. Further, a computer readable recording medium in which such a computer program is stored can be provided. Examples of the recording medium include a magnetic disk, an optical disk, a magneto-optical disk, and a flash memory. Also, the computer program may be distributed, for example, via a network, without using a recording medium.

### (4. Example of display screen)

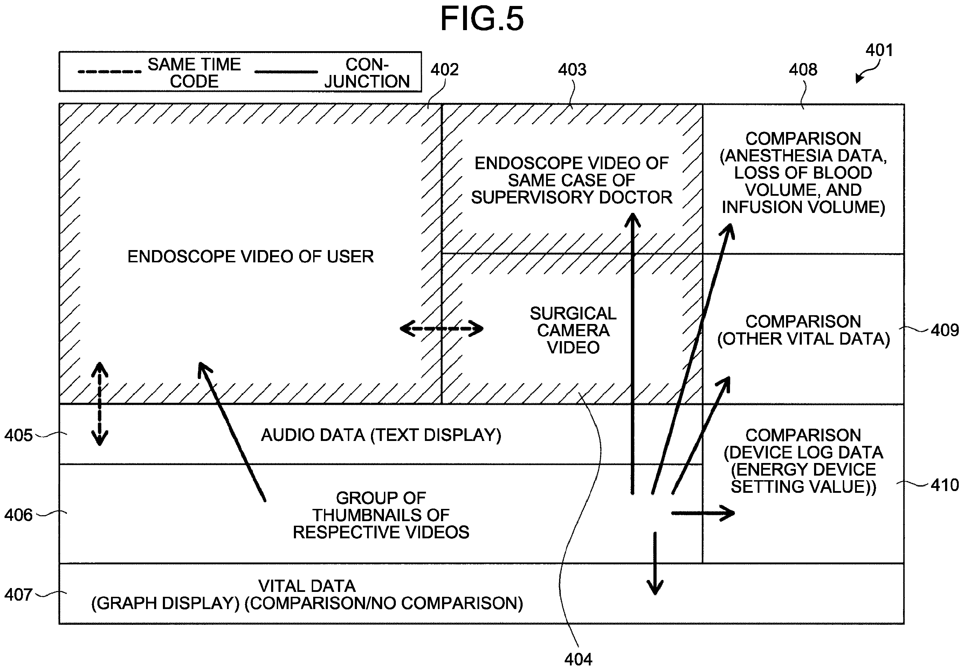

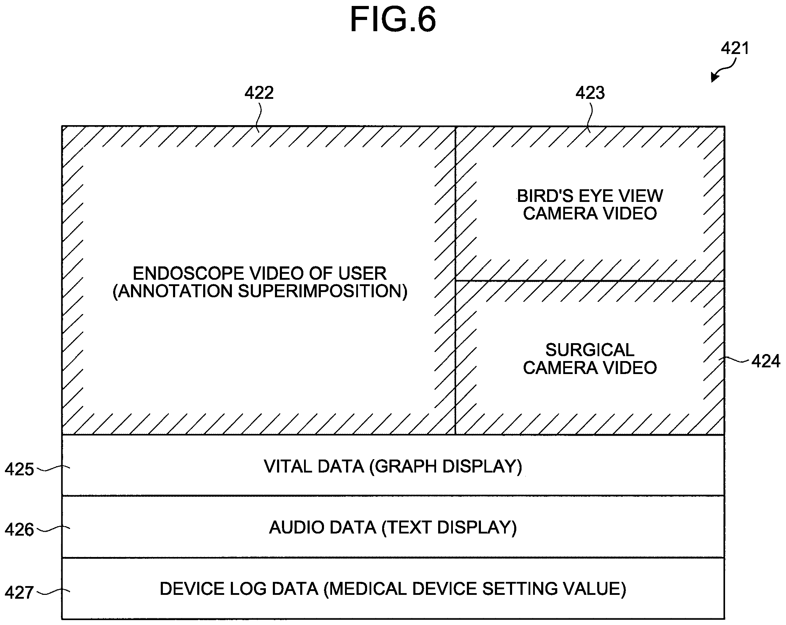

Several examples of the display screen in the terminal will be described with reference to FIGS. 5 and 6. FIG. 5 is a diagram illustrating an example of the display screen of the terminal on which the surgical data is displayed. In the example illustrated in FIG. 5, a display screen 401 is divided into nine areas. An endoscope video in the surgery performed by the user is displayed in the largest area 402. An endoscope video and a surgical camera video in the surgery of the same case performed by the supervisory doctor are displayed in areas 403 and 404 next to the area 402, respectively. The user can easily compare his/her technique with the technique of the supervisory doctor as his or her endoscope video and the endoscope video of the supervisory doctor are displayed side by side. Also, since his/her own endoscope video and the surgical camera video are displayed side by side, the user can simultaneously check a motion of the treatment tool in the body cavity and a motion of the treatment tool outside the body cavity.

At this time, the endoscope video and the surgical camera video of the user are video data acquired with the same time code in the same surgery. The endoscope video and the surgical camera video are played back on the display screen 401 with the same time code. On the other hand, the endoscope video of the supervisory doctor is a video acquired in a different surgery from the endoscope video of the user, thus it has a different time code from that of the endoscope video of the user. In this regard, in the present embodiment, the user can adjust a playback start position of the endoscope video of the supervisory doctor manually. Accordingly, it is possible to make, for example, an adjustment of playing back the scene of the same technique at the same time for the endoscope video of the user and the endoscope video of the supervisory doctor which are acquired with different time codes, and it is possible to compare the endoscope video of the user with the endoscope video of the supervisory doctor. The playback start position of the endoscope video of the supervisory doctor can be adjusted using a group of thumbnail images to be described later.

An area 405 in which audio data is displayed in the form of text is provided below the areas 402, 403, and 404. In the area 405, audio data acquired at the same time code as the endoscope video of the user is displayed in the form of text. Since the audio data includes verbal instructions and the like by the supervisory doctor, the audio data may be useful data when the user looks back at his/her own surgery through the endoscope video. Note that, there may be a situation in which no voice is unable to be output depending on an environment in which the display screen 401 is referred to, and in this case, it is very useful if the audio data is displayed in the form of text.

An area 406 in which a group of thumbnail images of videos displayed in the areas 402, 403, and 404 is displayed is provided below the area 405. In the area 406, thumbnail images of videos divided at predetermined time intervals are arranged and displayed chronologically for each video using a horizontal direction as a time direction. If one thumbnail image in a group of thumbnail images is selected by the user, a video corresponding to the thumbnail image is played back in the areas 402, 403, and 404 from the time corresponding to the thumbnail image. In other words, the user can select the playback start position of the video by selecting the thumbnail image. Since the playback position of each video is appropriately adjusted using such a group of thumbnail images, the user can perform, for example, an adjustment of simultaneously playing back the scene for the same technique for the endoscope video of the user and the endoscope video of the supervisory doctor.

Further, information specifying the scene of surgery may be displayed superimposed on each thumbnail image. By referring to the information, the user can select the thumbnail image indicating a desired playback position with a higher degree of accuracy. The information may be, for example, a setting value of a device included in the device log data. For example, a setting value of output power of the energy device can be increased or decreased with the progress of surgery. Therefore, the setting value is suitable as information to be superimposed on the thumbnail image because it can be an index indicating the progress of surgery. Of course, setting values of other medical devices may be used. At this time, as described above, since the thumbnail image is displayed at predetermined time intervals, the value displayed in a superimposed manner may be a setting value at a time corresponding to the thumbnail image or may be a maximum value, a minimum value, an average value, or a median value of the setting value in the corresponding period between the thumbnail images.

Also, each thumbnail image arranged in the time direction may be displayed with the same size or only a specific thumbnail image may be expanded and displayed in the time direction. For example, when a thumbnail image at a time at which some events occur during surgery is enlarged and displayed, the user can select the thumbnail image indicating a desired playback position with a higher degree of accuracy. The thumbnail image to be enlarged may be selected by the user or may be decided automatically on the basis of the device log data, the vital data, or the like. The decision criterion is appropriately decided by the user as a criterion indicating that an event has occurred at the time of surgery, for example, when the setting value of the medical device included in the device log data exceeds or falls below a threshold value.

An area 407 in which the vital data is displayed is provided below the area 406. In the area 407, any data included in the vital data is displayed as a graph. Here, in the area 407, the vital data corresponding to the endoscope video of the user (that is, the vital data acquired at the same as with the endoscope video data of the user) is displayed with the same time code as that of the endoscope video. In other words, in a case in which the user selects the thumbnail image in the area 406, the endoscope video of the user is played back from the time corresponding to that thumbnail image in conjunction with the thumbnail image, and the time change of the vital data from that time is displayed in the form of a graph. In the area 407, the vital data corresponding to the endoscopic video of the supervisory doctor may be displayed at the same time. The vital data may also be displayed at the same time code as the endoscope video of the supervisory doctor in conjunction with the selection of the thumbnail image. Accordingly, the user can simultaneously compare the endoscope video with the endoscope video of the supervisory doctor while comparing the vital data at that time in the area 407. Therefore, it is possible to compare the invasion given to patients in the surgery for the same case.

Further, areas 408, 409, and 410 for comparing the vital data, the anesthesia data, and the device log data corresponding to the endoscope video of the user with the vital data, the anesthesia data, and the device log data corresponding to the endoscope video of the supervisory doctor are further provided in the display screen 401. In the illustrated example, the anesthesia data and data of the loss of blood volume and the infusion volume included in the vital data are compared and displayed in the area 408, other data included in the vital data is compared and displayed in the area 409, and the setting value of the energy device in the device log data is compared and displayed in the area 410. The data displayed in the areas 408, 409, and 410 can also be displayed in conjunction with the selection of the thumbnail image with the same time code as the endoscope video of the user and the same time code as the endoscope video of the supervisory doctor. With the display in the areas 408, 409, and 410, it is possible to compare in more detail the invasion given to the patient.

In FIG. 5, for the sake of convenience of description, arrows illustrating the "same time code" and "conjunction" are illustrated, but it should be noted that the arrows are actually not displayed on the display screen.

FIG. 6 is a diagram illustrating another example of the display screen of the terminal on which the surgical data is displayed. In the example illustrated in FIG. 6, a display screen 421 is divided into six areas. The endoscope video of the user is displayed in the largest area 422, similarly to the example illustrated in FIG. 5. However, in the example illustrated in FIG. 6, unlike the example illustrated in FIG. 5, the endoscope video in which the annotation data is superimposed is displayed in the area 422. Since such annotation data is displayed in a superimposed manner, it is expected that review effects are further enhanced when a doctor who has been taught looks back at the endoscope video of his/her own surgery later.

A bird's eye view camera video and a surgical camera video are displayed in areas 423 and 424 next to the area 422, respectively. The bird's eye view camera video and the surgical camera video are displayed with the same time code as the endoscope video of the user. Accordingly, it is possible to look back at a situation of surgery by a plurality of videos in a plurality of ways.

An area 425 in which the vital data is displayed in the form of a graph, an area 426 in which the audio data is displayed in the form of a text, and an area 427 in which the device log data (the setting value of the medical device) is displayed are provided below the areas 422, 423, and 424. The vital data, the audio data, and the device log data are displayed with the same time code as the endoscope video of the user. As such data is displayed, it is possible to look back at influence of their own treatment on the patient, content of the verbal instructions from the supervisory doctor, content of conversations of a team, or the like while synchronizing with the endoscope video, and it can be expected that educational effects are further enhanced.

The several examples of the display screen in the terminal have been described above. The display screen is not limited to those illustrated in FIG. 5 and FIG. 6, and the surgical data and the display screen layout to be displayed on the display screen can be appropriately selected by the user. Further, the display screens of the surgical data described above may be displayed for the display screens of a plurality of terminals. In this case, the same display screen may be displayed in the respective terminals, or different display screens may be displayed in the respective terminals. In the former case, the index server 201 simultaneously outputs the generated presentation data to a plurality of terminals. In the latter case, the index server 201 generates a plurality of pieces of presentation data corresponding to a plurality of display screens and outputs them to a plurality of terminals. For example, in a case in which a plurality of doctors checks the same surgical data in their terminal at a conference or the like, the same display screen can be displayed in the respective terminals. Alternatively, in a case in which it is desired to check the video data on a larger screen, display screens related to different pieces of video data may be displayed in the two terminals arranged side by side.

### (5. Medical information management method)

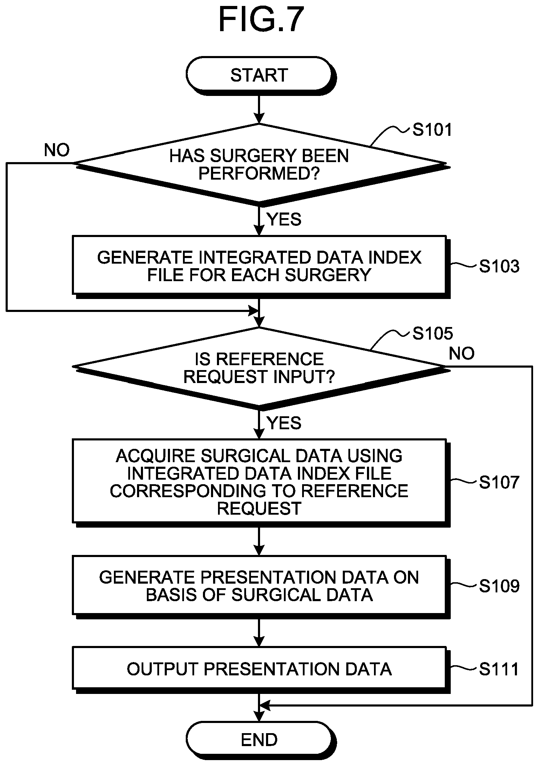

A processing procedure of an information processing method (a medical information management method) executed by the index server 201 described above will be described. FIG. 7 is a flow chart illustrating an example of the processing procedure of the medical information management method according to the present embodiment. Processes illustrated in FIG. 7 corresponds to the processes executed by the index server 201 illustrated in FIGS. 1, 3, and 4. The processes illustrated in FIG. 7 are executed as the processor of the index server 201 executes arithmetic processing in accordance with a predetermined program. The details of the processes illustrated in FIG. 7 have been described above when the functions of the index server 201 are described, and in the following description of the processes illustrated in FIG. 7, the overviews of the processes will be described, and detailed description thereof is omitted.

Referring to FIG. 7, in the medical information management method according to the present embodiment, first, it is decided whether or not surgery has been performed (Step S101).

In a case in which it is determined in Step S101 that the surgery has been performed, the process proceeds to Step S103. In Step S103, the integrated data index file for the surgery is generated. If the process in Step S103 ends, the process proceeds to Step S105. The process of Steps S101 and S103 corresponds to the process executed by the integrated data index file generating unit 202 illustrated in FIG. 4.

In a case in which it is determined in Step S101 that the surgery has not been performed, the process of Step S103 is skipped, and the process proceeds to Step S105. In Step S105, it is determined whether or not the reference request for the surgical data is input.

In a case in which it is determined in Step S105 that the reference request is not input, a series of processes ends.

On the other hand, in a case in which it is determined in Step S105 that the reference request is input, the process proceeds to Step S107. In Step S107, the surgical data is acquired using the integrated data index file corresponding to the reference request. In a case in which the patient information is also requested in the reference request, the patient information may be acquired together using the corresponding integrated data index file. The process of Step S101 and Step S103 corresponds to the process executed by the data acquiring unit 203 illustrated in FIG. 4.

Then, the presentation data is generated on the basis of the acquired surgical data (Step S109). Then, the generated presentation data is output to the terminal (Step S111), and a series of processes ends. The process of Steps S109 and S111 corresponds to the process executed by the presentation data generating unit 204 illustrated in FIG. 4.

### (6. Hardware configuration)

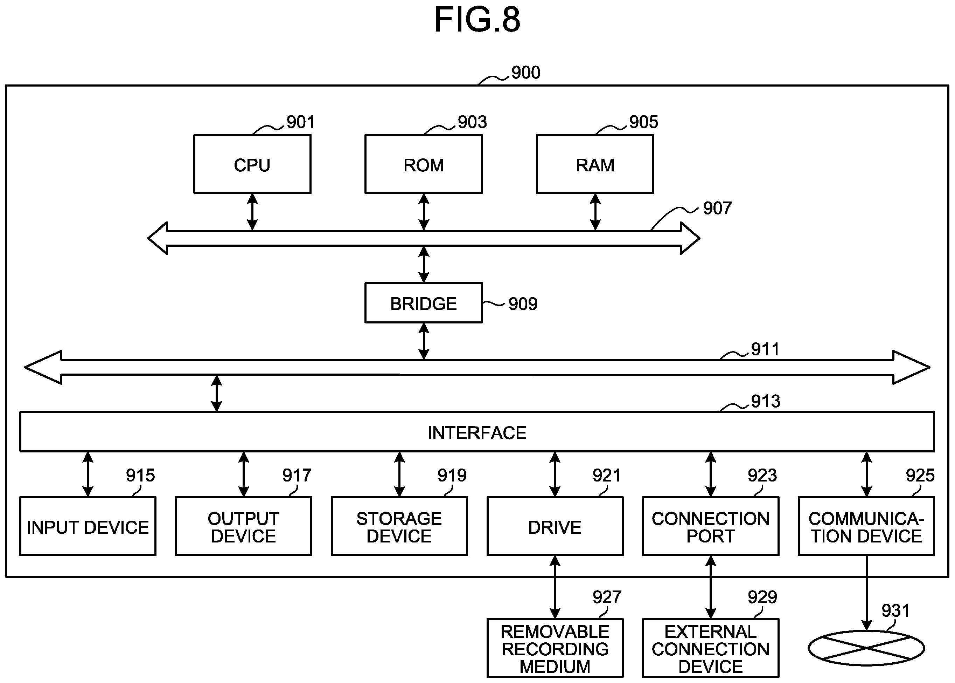

A hardware configuration of the medical information management apparatus according to the present embodiment will be described with reference to FIG. 8. FIG. 8 is a block diagram illustrating an example of a hardware configuration of the medical information management apparatus according to the present embodiment. An illustrated medical information management apparatus 900 can implement the index server 201 in the above embodiment.

The medical information management apparatus 900 includes a CPU 901, a read only memory (ROM) 903, and a random-access memory (RAM) 905. The medical information management apparatus 900 may include a host bus 907, a bridge 909, an external bus 911, an interface 913, an input device 915, an output device 917, a storage device 919, a drive 921, a connection port 923, and a communication device 925. The medical information management apparatus 900 may include a processing circuit such as a DSP or an application specific integrated circuit (ASIC) instead of or in addition to the CPU 901.

The CPU 901 functions as an arithmetic processing device and a control device, and controls the overall or partial operation of the medical information management apparatus 900 in accordance with various types of programs recorded in the ROM 903, the RAM 905, the storage device 919, or a removable recording medium 927. The ROM 903 stores programs, operation parameters, and the like used by the CPU 901. The RAM 905 temporarily stores programs used in execution of the CPU 901, parameters that appropriately change in execution thereof, and the like. The CPU 901, the ROM 903 and the RAM 905 are connected to one another via the host bus 907 constituted by an internal bus such as a CPU bus. Furthermore, the host bus 907 is connected to the external bus 911 such as a peripheral component interconnect/interface (PCI) bus via the bridge 909. The CPU 901 can configure, for example, the integrated data index file generating unit 202, the data acquiring unit 203, and the presentation data generating unit 204 illustrated in FIG. 4 in the present embodiment.

The input device 915 is a device operated by the user such as, for example, a mouse, a keyboard, a touch panel, a button, a switch, or a lever. The input device 915 may be, for example, a remote-control device using infrared rays or other radio waves or may be an external connection device 929 such as a mobile phone corresponding to the operation of the medical information management apparatus 900. The input device 915 includes an input control circuit that generates an input signal on the basis of information input by the user and outputs the signal to the CPU 901. The user inputs various types of data to the medical information management apparatus 900 and instructs a processing operation by operating the input device 915.

The output device 917 includes a device that can notify the user of acquired information visually or aurally. The output device 917 may be, for example, a display device such as a liquid crystal display device, a plasma display device, an organic EL display device, a lamp, a projector, or an illumination, an audio output device such as a speaker or a headphone, and a printer. The output device 917 outputs a result obtained by a process in the medical information management apparatus 900 as a video such as a text or an image or outputs it as an audio such as a voice or a sound.

The storage device 919 is a data storage device that functions as a storage unit of the medical information management apparatus 900. The storage device 919 is constituted by, for example, a magnetic storage unit device such as an HDD, a semiconductor storage device, an optical storage device, a magneto-optical storage device, or the like. The storage device 919 stores programs executed by the CPU 901, various types of data, and various types of data acquired from the outside. In the present embodiment, for example, the storage device 919 may constitute the storage unit 205 illustrated in FIG. 4.

The drive 921 is a reader/writer for the removable recording medium 927 such as a magnetic disk, an optical disk, a magneto-optical disk, or a semiconductor memory and is installed in or externally attached to the medical information management apparatus 900. The drive 921 reads information recorded in the loaded removable recording medium 927 and outputs the information to the RAM 905. Further, the drive 921 writes information in the loaded removable recording medium 927. The drive 921 can read various types of information to be processed in the medical information management apparatus 900 from the removable recording medium 927 or write the information in the removable recording medium 927.

The connection port 923 is a port for directly connecting a device to the medical information management apparatus 900. The connection port 923 may be, for example, a universal serial bus (USB) port, an IEEE 1394 port, a small computer system interface (SCSI) port, or the like. Further, the connection port 923 may be an RS-232C port, an optical audio terminal, a high-definition multimedia interface (HDMI) (registered trademark) port, or the like. When the external connection device 929 is connected to the connection port 923, various types of data can be exchanged between the medical information management apparatus 900 and the external connection device 929. Various types of information processed in the medical information management apparatus 900 can be transmitted to and received from the external connection device 929 via the connection port 923.

The communication device 925 is, for example, a communication interface constituted by a communication device or the like for connecting to a communication network 931. The communication device 925 may be, for example, a communication card for a wired or wireless local area network (LAN), Bluetooth (registered trademark), or WUSB (Wireless USB). Also, the communication device 925 may be an optical communication router, an asymmetric digital subscriber line (ADSL) router, various types of communication modems, or the like. For example, the communication device 925 performs transmission and reception of signals or the like using a predetermined protocol such as TCP/IP with the Internet or other communication devices. Also, the communication network 931 connected to the communication device 925 is a network connected in a wired or wireless manner, and is, for example, the Internet, a home LAN, infrared communication, radio wave communication, satellite communication, or the like. The communication device 925 can performs transmission and reception of various types of information processed in the medical information management apparatus 900 with other external devices via the communication network 931.

The example of the hardware configuration of the medical information management apparatus 900 has been described above. Each of the components described above may be constituted using a general-purpose member or may be constituted by hardware specialized for the function of each component. Such a configuration may be changed appropriately depending on the level of technology at the time of implementation.

### (7. Supplement)

Although the exemplary embodiment of the present disclosure has been described in detail with reference to the appended drawings, the technical scope of the present disclosure is not limited to such an example. It is obvious that those skilled in the technical field of the present disclosure can conceive of various modifications or alterations within the scope of the technical idea described in claims set forth below, and it is understood that it, of course, belongs to the technical scope of the present disclosure.

For example, in the above embodiment, the presentation data is generated in the index server 201, and the process of outputting the presentation data is performed in the terminal, but the present disclosure is not limited to such an example. For example, the index server 201 may have only a function of generating the integrated data index file and a function of acquiring the surgical data using the integrated data index file in accordance with the reference request. In this case, the index server 201 transmits the acquired surgical data to the terminal. Then, the terminal may generate the presentation data using the surgical data and output the presentation data. Alternatively, a part of the presentation data generation process may be performed by the index server 201, and the remaining process may be performed in the terminal. However, since the surgical data can be a huge amount of data, it is likely that it is difficult to process the surgical data in the terminal in a case in which the information processing capability of the terminal is low. Therefore, in the present disclosure, the configuration according to the above-described embodiment in which the presentation data is generated in the index server 201 having higher information processing capacity is considered to be more desirable.

Also, in the above embodiment, in a case in which the video data acquired in different surgeries are played back together, the process of aligning the playback start position is performed by the operation of selecting the thumbnail image by the user, but the present disclosure is not limited to the example. The process of aligning the playback start position may be performed automatically when the presentation data is generated by the index server 201. For example, the playback start position may be aligned by analyzing two pieces of video data using an image analysis technique, specifying a type of organ or treatment tool in the video, and detecting the progress of the surgery in the two pieces of video data. Alternatively, the playback start position may be aligned by analyzing the time change of the setting value of the medical device with reference to the device log data and detecting the progress of the surgery in the two pieces of video data.

Also, the effects described in this specification are merely explanatory or illustrative and not limiting. In other words, the technology according to the present disclosure can have other effects apparent to those skilled in the art from the description of this specification in addition to or instead of the effects described above.

The following configurations also belong to the technical scope of the present disclosure.
(1) A medical information management apparatus, including:
   an integrated data index file generating unit that generates an integrated data index file in which an index indicating a storage location of surgical data which is various types of data acquired during surgery is integrated with an index indicating a storage location of patient information of a patient which is a subject of the surgery for each surgery.
(2) The medical information management apparatus according to (1), further including:
   a data acquiring unit that accesses, in a case in which a reference request for referring to the surgical data is input, a recording device in which the surgical data designated by the reference request is recorded using the integrated data index file corresponding to the surgical data designated by the reference request and acquires the surgical data designated by the reference request.
(3) The medical information management apparatus according to (2), further including:
   a presentation data generating unit that generates presentation data to be output in a terminal of a user who made the reference request using the surgical data acquired by the data acquiring unit.
(4) The medical information management apparatus according to (3), in which the surgical data includes video data representing a situation of the surgery,
   the presentation data includes display screen data displayed in the terminal, and
   in the display screen data, the video data and at least one of other data included in the surgical data is displayed with the same time code.
(5) The medical information management apparatus according to (4), in which the video data further includes at least one of endoscope video data captured by an endoscope, bird's eye view camera video data captured by a bird's eye view camera that captures a bird's eye view image in an operating room, and surgical camera video data captured by a surgical camera that images a surgical field, and
   in the display screen data, at least two of the endoscope video data, the bird's eye view camera video data, and the surgical camera video data are displayed with the same time code.
(6) The medical information management apparatus according to (4) or (5), in which the surgical data further includes at least one of vital data of the patient, anesthesia data which is information related to anesthesia for the patient, and device log data which is log data of a medical device, and
   in the display screen data, the video data and at least one of the vital data, the anesthesia data, and the device log data are displayed with the same time code.
(7) The medical information management apparatus according to any one of (4) to (6), in which the surgical data further includes audio data in the operating room, and
   in the display screen data, the video data and a text related to the audio data are displayed with the same time code.
(8) The medical information management apparatus according to any one of (4) to (7), in which the surgical data further includes annotation data given to the video data displayed during the surgery by a supervisory doctor, and
   in the display screen data, the video data and the annotation data are displayed with the same time code.
(9) The medical information management apparatus according to any one of (3) to (8), in which the presentation data includes display screen data displayed in the terminal, and
   in the display screen data, the patient information is displayed together with the surgical data.
(10) The medical information management apparatus according to any one of (3) to (9), in which the presentation data includes display screen data displayed in the terminal, and
   in the display screen data, the surgical data in two different surgeries are displayed together.
(11) The medical information management apparatus according to (10), in which, as the surgical data in two different surgeries, video data in surgery performed by each of different surgeons is displayed.
(12) The medical information management apparatus according to (10) or (11), in which, as the surgical data in two different surgeries, at least one of vital data of the patient, anesthesia data which is information related to anesthesia for the patient, and device log data which is log data of a medical device in surgeries performed by different surgeons is compared and displayed.
(13) The medical information management apparatus according to any one of (3) to (12), in which the surgical data includes video data representing a situation of the surgery,
   the presentation data includes display screen data displayed in the terminal, and
   in the display screen data, thumbnail images at respective times in which the video data is divided at predetermined time intervals are displayed side by side in a time direction along with the video data, and the video data is played back from a time corresponding to the selected thumbnail image.
(14) The medical information management apparatus according to (13), in which the surgical data further includes at least one of vital data of the patient, anesthesia data which is information related to anesthesia for the patient, and device log data which is log data of a medical device, and
   at least one of the vital data, the anesthesia data, and the device log data is displayed superimposed on the thumbnail image.
(15) The medical information management apparatus according to (14), in which at least one value of the vital data, the anesthesia data, and the device log data at a time corresponding to the thumbnail image is displayed superimposed on the thumbnail image.
(16) The medical information management apparatus according to (14), in which a minimum value, a maximum value, an average value, or a median value of at least one of the vital data, the anesthesia data, and the device log data in a corresponding period between the thumbnail images arranged in the time direction is displayed superimposed on the thumbnail image.
(17) The medical information management apparatus according to any one of (13) to (16), in which some of a plurality of thumbnail images arranged in the time direction are expanded and displayed in a time direction.
(18) The medical information management apparatus according to any one of (3) to (17), in which the presentation data generating unit extracts event information related to an event associated with surgery detected from the surgical data on the basis of an instruction of the user and generates the presentation data.
(19) A medical information management method, including:
   generating, by a processor, an integrated data index file in which an index indicating a storage location of surgical data which is various types of data acquired during surgery is integrated with an index indicating a storage location of patient information of a patient which is a subject of the surgery for each surgery.
(20) A medical information management system, including:
   a recording device that records and stores surgical data which is various types of data acquired during surgery during the surgery;
   a medical information management apparatus that manages the surgical data in an integrated manner; and
   a terminal that displays the surgical data,
   in which the medical information management apparatus includes
   an integrated data index file generating unit that generates an integrated data index file in which an index indicating a storage location of surgical data which is various types of data acquired during surgery is integrated with an index indicating a storage location of patient information of a patient which is a subject of the surgery for each surgery;
   a data acquiring unit that accesses, in a case in which a reference request for referring to the surgical data is input, a recording device in which the surgical data designated by the reference request is recorded using the integrated data index file corresponding to the surgical data designated by the reference request and acquires the surgical data designated by the reference request; and
   a presentation data generating unit that generates presentation data to be output in a terminal of a user who made the reference request using the surgical data acquired by the data acquiring unit.

### Reference Signs List

- 1: SURGICAL DATA RECORDING SYSTEM
- 2: SURGICAL DATA REFERENCE SYSTEM
- 101: MEDICAL CONTROLLER
- 111: BIRD'S EYE VIEW CAMERA
- 112: SURGICAL CAMERA
- 113: ENDOSCOPE DEVICE/MEDICAL DEVICE
- 114: EXTERNAL INPUT TERMINAL
- 115: INTERCOM
- 116: BIOMONITOR
- 117: ANESTHESIA DEVICE
- 121: RECORDER
- 122: MOVING IMAGE SERVER
- 123: DEVICE LOG DATA SERVER
- 124: ANESTHESIOLOGY SERVER
- 131: CEILING-INSTALLED MONITOR
- 132: WALL MONITOR
- 133: PRINTER
- 141: ELECTRONIC MEDICAL CHART SERVER
- 142: DICOM SERVER
- 151: TOUCH PANEL
- 152: TOUCH PANEL INTERFACE
- 161: SWITCHER
- 162: SWITCHER CONTROL DEVICE
- 171: ILLUMINATING DEVICE
- 181: CONVERTER
- 201: INDEX SERVER
- 202: INTEGRATED DATA INDEX FILE GENERATING UNIT
- 203: DATA ACQUIRING UNIT
- 204: PRESENTATION DATA GENERATING UNIT
- 205: STORAGE UNIT
- 301: CONFERENCE MONITOR
- 302: MEDICAL OFFICE PC
- 303: TABLET PC
- 304: SETUP BOX

## Claims

1. A medical information management apparatus comprising:
an integrated data index file generating unit that generates an integrated data index file in which an index indicating a storage location of surgical data which is various types of data acquired during surgery is integrated, for each surgery, with an index indicating a storage location of patient information of a patient which is a subject of the surgery.

2. The medical information management apparatus according to claim 1, further comprising:
a data acquiring unit that accesses, in a case in which a reference request for referring to the surgical data is input, a recording device in which the surgical data designated by the reference request is recorded using the integrated data index file corresponding to the surgical data designated by the reference request and acquires the surgical data designated by the reference request.

3. The medical information management apparatus according to claim 2, further comprising:
a presentation data generating unit that generates presentation data to be output in a terminal of a user who made the reference request using the surgical data acquired by the data acquiring unit.

4. The medical information management apparatus according to claim 3, wherein the surgical data includes video data representing a situation of the surgery,
the presentation data includes display screen data displayed in the terminal, and
in the display screen data, the video data and at least one of other data included in the surgical data is displayed with the same time code.

5. The medical information management apparatus according to claim 4, wherein the video data further includes at least one of endoscope video data captured by an endoscope, bird's eye view camera video data captured by a bird's eye view camera that captures a bird's eye view image in an operating room, and surgical camera video data captured by a surgical camera that images a surgical field, and
in the display screen data, at least two of the endoscope video data, the bird's eye view camera video data, and the surgical camera video data are displayed with the same time code.

6. The medical information management apparatus according to claim 4, wherein the surgical data further includes at least one of vital data of the patient, anesthesia data which is information related to anesthesia for the patient, and device log data which is log data of a medical device, and
in the display screen data, the video data and at least one of the vital data, the anesthesia data, and the device log data are displayed with the same time code.

7. The medical information management apparatus according to claim 4, wherein the surgical data further includes audio data in the operating room, and
in the display screen data, the video data and a text related to the audio data are displayed with the same time code.

8. The medical information management apparatus according to claim 4, wherein the surgical data further includes annotation data given to the video data displayed during the surgery by a supervisory doctor, and
in the display screen data, the video data and the annotation data are displayed with the same time code.

9. The medical information management apparatus according to claim 3, wherein the presentation data includes display screen data displayed in the terminal, and
in the display screen data, the patient information is displayed together with the surgical data.

10. The medical information management apparatus according to claim 3, wherein the presentation data includes display screen data displayed in the terminal, and
in the display screen data, the surgical data in two different surgeries are displayed together.

11. The medical information management apparatus according to claim 10, wherein, as the surgical data in two different surgeries, video data in surgery performed by each of different surgeons is displayed.

12. The medical information management apparatus according to claim 10, wherein, as the surgical data in two different surgeries, at least one of vital data of the patient, anesthesia data which is information related to anesthesia for the patient, and device log data which is log data of a medical device in surgeries performed by different surgeons is compared and displayed.

13. The medical information management apparatus according to claim 3, wherein the surgical data includes video data representing a situation of the surgery,
the presentation data includes display screen data displayed in the terminal, and
in the display screen data, thumbnail images at respective times in which the video data is divided at predetermined time intervals are displayed side by side in a time direction along with the video data, and the video data is played back from a time corresponding to the selected thumbnail image.

14. The medical information management apparatus according to claim 13, wherein the surgical data further includes at least one of vital data of the patient, anesthesia data which is information related to anesthesia for the patient, and device log data which is log data of a medical device, and
at least one of the vital data, the anesthesia data, and the device log data is displayed superimposed on the thumbnail image.

15. The medical information management apparatus according to claim 14, wherein at least one value of the vital data, the anesthesia data, and the device log data at a time corresponding to the thumbnail image is displayed superimposed on the thumbnail image.

16. The medical information management apparatus according to claim 14, wherein a minimum value, a maximum value, an average value, or a median value of at least one of the vital data, the anesthesia data, and the device log data in a corresponding period between the thumbnail images arranged in the time direction is displayed superimposed on the thumbnail image.

17. The medical information management apparatus according to claim 13, wherein some of a plurality of thumbnail images arranged in the time direction are expanded and displayed in a time direction.

18. The medical information management apparatus according to claim 3, wherein the presentation data generating unit extracts event information related to an event associated with surgery detected from the surgical data on the basis of an instruction of the user and generates the presentation data.

19. A medical information management method, comprising:
generating, by a processor, an integrated data index file in which an index indicating a storage location of surgical data which is various types of data acquired during surgery is integrated, for each surgery, with an index indicating a storage location of patient information of a patient which is a subject of the surgery.

20. A medical information management system, comprising:
a recording device that records and stores surgical data which is various types of data acquired during surgery during the surgery;
a medical information management apparatus that manages the surgical data in an integrated manner; and
a terminal that displays the surgical data,
wherein the medical information management apparatus includes:
an integrated data index file generating unit that generates an integrated data index file in which an index indicating a storage location of surgical data which is various types of data acquired during surgery is integrated, for each surgery, with an index indicating a storage location of patient information of a patient which is a subject of the surgery;
a data acquiring unit that accesses, in a case in which a reference request for referring to the surgical data is input, a recording device in which the surgical data designated by the reference request is recorded using the integrated data index file corresponding to the surgical data designated by the reference request and acquires the surgical data designated by the reference request; and
a presentation data generating unit that generates presentation data to be output in a terminal of a user who made the reference request using the surgical data acquired by the data acquiring unit.
